# EUROPÄISCHE PATENTSCHRIFT

(11) **EP 3 058 877 B1**
(45) Veröffentlichungstag und Bekanntmachung des Hinweises auf die Patenterteilung: **30.01.2019**
(21) Anmeldenummer: 16156983.5
(22) Anmeldetag: 23.02.2016
(51) Int. Cl.: A61B 10/00

(54) **VORRICHTUNG ZUM TRANSPORT, ZUR UNTERSUCHUNG UND/ODER ZUR LAGERUNG EINER GEWEBEPROBE**
DEVICE FOR TRANSPORTING, EXAMINING AND/OR STORING A TISSUE SAMPLE
DISPOSITIF DE TRANSPORT, D'ANALYSE ET/OU DE STOCKAGE D'UN ECHANTILLON DE TISSU

(30) Priorität: 23.02.2015 DE 102015002109
(43) Veröffentlichungstag der Anmeldung: 24.08.2016
(73) Patentinhaber: FWB Kunststofftechnik GmbH, 66955 Pirmasens (DE)
(72) Erfinder: Huschmand Nia, Abdolhamid, 66953 Pirmasens (DE)
(74) Vertreter: Patentanwälte Bernhardt / Wolff Partnerschaft mbB

(56) Entgegenhaltungen:
- WO-A2-02/059571
- WO-A2-2006/092797
- DE-U1-202004 005 318
- US-A- 5 383 472
- US-A1- 2011 104 797

## Beschreibung

Die Erfindung betrifft eine Vorrichtung zum Transport, zur Untersuchung und/oder zur Lagerung einer Gewebeprobe, die eine Grundplatte, welche einen zumindest teilweise röntgentransparenten Abschnitt zur Aufnahme der Gewebeprobe umfasst und mit einer Markierung zur Anzeige der topografischen Orientierung der Gewebeprobe versehen ist, und zumindest ein Mittel zur Fixierung der Gewebeprobe auf dem Aufnahmeabschnitt aufweist, das derart eingerichtet ist, dass es sich unter Fixierung der Gewebeprobe lösbar an einem Rand oder außerhalb des Aufnahmeabschnitts an der Grundplatte befestigen lässt, wobei die Vorrichtung vorgesehen ist derart, dass eine Position, in der sich das Fixierungsmittel unter Fixierung der Gewebeprobe an der Grundplatte lösbar befestigen lässt, frei wählbar ist und das Fixierungsmittel zu seiner Befestigung an der Grundplatte mit einer Befestigungseinrichtung versehen ist, mittels derer sich das Fixierungsmittel lösbar in einer Halteposition zum Halten der Gewebeprobe stecken und/oder in der Halteposition einrasten lässt.

Aus der WO 02/059571 beschreibt eine solche Vorrichtung, die zwei kastenartig geformte Halteteile umfasst, die derart ineinandergesteckt werden können, dass sie einen würfelförmigen Raum bilden, in denen Gewebe zwischen den Halteteilen festgehalten wird.

Weitere Vorrichtungen zum Transport, zur Untersuchung und/oder zur Lagerung einer Gewebeprobe gehen aus DE 20 2004 005 318 U1, US 5,383,472, WO 2006/092797 A2 und US 2011/0104797 A1 hervor.

Chirurgisch entnommenes Tumorgewebe wird durch den Pathologen mikroskopisch untersucht um Art, Gut- oder Bösartigkeit, biologische Eigenschaften und die Größe des Tumors zu bestimmen. Auch die vollständige Entfernung des Tumors mit einem schmalen Rand von gesundem Gewebe (freier Resektionsrand) wird mikroskopisch überprüft. Die Befunde einer mikroskopischen Untersuchung liegen in der Regel einige Tage nach der Operation vor, weil das Gewebe erst nach vielen weiteren Schritten (z.B. Fixierung in Formalin, Zuschnitt und Färbung) unter dem Mikroskop beurteilt werden kann. Wurde ein Tumor bei der Erstoperation nicht oder nicht vollständig oder nicht mit einem als ausreichend definierten freien Resektionsrand entfernt, wird in der Regel eine zweite Operation durchgeführt, um das Operationsergebnis durch eine sog. "Nachresektion" zu korrigieren. Zu diesem Zweck ist es erforderlich, dass die korrekte Orientierung der entnommenen Gewebeprobe innerhalb des Körpers zweifelsfrei erkennbar dokumentiert wird. Sonst kann der Pathologe zwar gegebenenfalls die mangelhafte Operation beschreiben, aber nicht angeben, wo im Operationsgebiet der ersten Operation eine Korrektur (Nachresektion) notwendig ist. Besser wäre, das Gewebe noch während der Erstoperation zu überprüfen, um gegebenenfalls die notwendigen Korrekturen bereits bei der Erstoperation vornehmen zu können. Hierfür kann das Gewebe einer schnellen mikroskopischen Untersuchung (Schnellschnitt-Untersuchung) zugeführt werden. Die Schnellschnitt-Untersuchung setzt die unmittelbare Verfügbarkeit eines erfahrenen, entsprechend qualifizierten Pathologen voraus und ist deshalb nicht in jedem Krankenhaus möglich. Außerdem gilt die "Schnellschnitt Untersuchung" eines Tumors für die Beurteilung der Resektionsränder als nur eingeschränkt geeignet.

Das Problem kann gelöst werden, wenn statt der schnellen und somit weniger zuverlässigen mikroskopischen Untersuchung, das Gewebe einer radiologischen oder sonographischen (durch Ultraschall) Untersuchung zugeführt wird. Voraussetzung für die Kommunikation zwischen Operateur, Radiologen und Pathologen ist hierbei eine zuverlässige, topographisch korrekte Fixierung des entnommenen Gewebes, da es praktisch unmöglich ist, ein lose transportiertes Gewebe in der ursprünglichen im Körper vorliegenden Orientierung zu präsentieren.
Unabhängig davon, welches der genannten Untersuchungsverfahren eingesetzt wird, ist es wichtig das Gewebe noch am Operationstisch durch den Operateur so fixieren zu lassen, dass die topografische Orientierung des Gewebes im Körper festgehalten wird und später nachvollziehbar ist.

Um entnommene Gewebeprobe hinsichtlich ihrer Orientierung im Körper zu kennzeichnen, werden verschiedene Verfahren eingesetzt. Eine einheitliche Methode gibt es nicht. Üblich sind Farb- und Fadenmarkierungen. Hierbei kommt es regelmäßig zu Übermittlungsfehlern und Fehlinterpretationen, die zum Nachteil der Patienten sein können. Neben der Farb- und Fadenmarkierung, wird die Befestigung an Korkplatten mittels Stahlkanülen, die normalerweise für Injektionszwecke verwendet werden, und die Befestigung auf Platten aus PUR-Hartschaum mittels speziell hierfür entwickelten Pins praktiziert. Während Korkplatten und Injektionsnadel als völlig ungeeignet und vor allem wegen Stichverletzungen als zu gefährlich abgelehnt werden müssen, hat die Befestigung auf PUR-Hartschaumplatten mittels spezieller Pins, wie aus der EP 2 303 138 B1 bekannt, die Gefahr von Stichverletzungen deutlich reduziert. Auch Übermittlungsfehler und Fehlinterpretationen sind bei dem letztgenannten Verfahren bei korrekter Handhabung viel seltener, weil die PUR-Hartschaumplatten mit festen Orientierungsmarkern versehen sind, die sowohl optisch lesbar als auch im Röntgenbild sichtbar sind.
Die Befestigung auf Hartschaumplatten hat aber den Nachteil, dass die Unterseite der Gewebeprobe, d.h. die basale Fläche, nicht ausreichend mit Formalin in Kontakt kommt, das zur Lagerung auf die Gewebeprobe gegeben wird. Gewebeproben müssen vor der weiteren Verarbeitung in der Pathologie eine Zeitlang in Formalin gelagert werden, damit sie dann für die weiteren Schritte der feingeweblichen Untersuchungen vorbereitet sind. Die Pathologen nennen diesen Vorgang "Formalin-Fixierung". Unzureichende oder uneinheitliche Formalin-Fixierung der Gewebeproben erschwert die Untersuchungen. Ein weiteres Problem bei PUR-Hartschaumplatten sowohl bei dem Transport und der Lagerung als auch bei der Entsorgung ist das große Volumen der PUR-Platten. Auch die Produktion der Platten ist aufwendig, weil mehrere Produktions- und Montageschritte notwendig sind. Außerdem müssen bei den Vorrichtungen nach der EP 2 303 138 B1 für Gewebeproben der linken und der rechten Körperseite jeweils unterschiedlich konfigurierte PUR-Platten hergestellt werden. Diese müssen auch vor Ort getrennt gelagert werden. Zudem stellt sich die Bestellorganisation von Platten als problematisch dar, weil Kliniken jeweils überprüfen müssen, wie viele Platten für welche Körperseite bestellt werden müssen. Ein weiteres Problem der Anordnung nach der EP 2 303 138 B1 stellt das Anfertigen von Röntgenaufnahmen in cranial/caudalen Strahlengang. Hierfür muss die PUR Platte mit Hilfe eines zusätzlichen Ständers vertikal aufgestellt werden.

Aus US 4 993 056 A ist ein Tablett zum Transport und zur Aufbewahrung von Gewebeproben bekannt, bei dem die Gewebeprobe zwischen zwei Platten eines Tabletts zusammengepresst und festgeklemmt wird. Eine der beiden Platten trägt eine geschlossene röntgendichte Gitterstruktur auf der Oberfläche. Auch hier ist eine ausreichende Formalin-Fixierung nicht möglich, weil zwei Seiten der Gewebeprobe von den beiden Platten abgedeckt werden. Außerdem führt das Zusammenpressen einer Gewebeprobe zwischen zwei Platten dazu, dass der freie Resektionsrand in zwei Ebenen (oben und unten) fälschlicherweise kleiner und in den anderen vier Ebenen großer gemessen wird. Das Verfahren ist außerdem für einen längeren Transportweg ungeeignet, weil das Gewebe nicht dauerhaft zwischen den Platten fixiert bleibt.

Der Erfindung liegt die Aufgabe zugrunde, eine eingangs genannte Anordnung derart weiterzubilden, dass sich das entnommene Gewebe besser handhaben lässt.

Erfindungsgemäß wird diese Aufgabe dadurch gelöst, dass Fixierungsmittel einen über den Aufnahmeabschnitt ragenden Steg und ein an dem Steg angeordnetes, zum Aufnahmeabschnitt hin vorstehendes und zum Angriff an der Gewebeprobe vorgesehenes Haltemittel umfasst.

Vorteilhaft lässt sich das Fixierungsmittel zum Fixieren der Gewebeprobe so an der Grundplatte befestigen, dass es bei Untersuchung der Gewebeprobe, insbesondere bei Anfertigung einer Röntgenaufnahme, nicht oder nur wenig stört bzw. die Gewebeprobe im Röntgenbild nicht oder nur geringfügig überdeckt. Das entnommene Gewebe ist bei Anordnung an der erfindungsgemäßen Vorrichtung einer radiologischen und sonographischen Untersuchung so zugänglich, dass die Resektionsränder des Tumors topographisch korrekt und unverfälscht dargestellt und noch während der laufenden Operation ohne Schnellschnitt-Untersuchung gemessen und jeweils der entsprechenden Lokalisation im Körper des Patienten zugeordnet werden können.
Darüber hinaus lässt sich die Vorrichtung preiswert und mit geringem Volumen herstellen und kann für Gewebeproben sowohl der rechten als auch der linken Körperseite verwendet werden.

In einer Ausgestaltung der Erfindung ist die Vorrichtung derart vorgesehen, dass eine Position, in der sich das Fixierungsmittel außerhalb des Aufnahmeabschnitts an der Grundplatte befestigen lässt, stufenlos, wählbar ist.

Zweckmäßigerweise weist die Grundplatte eine Befestigungsschiene, an der sich das Fixierungsmittel befestigen lässt, auf. Die Befestigungsschiene ist vorzugsweise entlang des Randes der Grundplatte und/oder des Aufnahmeabschnitts gebildet.

Die genannte Befestigungseinrichtung ist zweckmäßigerweise zum Einrasten an der Befestigungsschiene vorgesehen. Sie kann dazu mit einer von einem der Ränder der Grundplatte vorstehenden Leiste versehen sein, und die Befestigungseinrichtung eine Raste umfassen, die bei Anordnung des Fixierungsmittels in der Fixierungsposition die Leiste hintergreift.

In einer weiteren Ausgestaltung der Erfindung ist der genannte, über den Aufnahmeabschnitt ragende Steg mit zumindest einem mit der Befestigungseinrichtung versehenen Schenkel verbunden ist.

Zweckmäßigerweise weist das Fixierungsmittel zumindest ein von dem Steg zum Aufnahmeabschnitt hin vorstehendes, zum Angriff an der Gewebeprobe vorgesehenes Haltemittel, vorzugsweise einen Stift oder/und eine Nadel, auf. In einer Ausführungsform der Erfindung lässt sich das Haltemittel an dem Steg in verschiedenen Positionen anordnen, vorzugsweise an dem Steg anstecken oder dort einrasten. Vorteilhaft lässt sich das Fixierungsmittel derart an der Grundplatte befestigen, dass lediglich das Haltemittel, nicht jedoch der Steg und ggf. der Schenkel und die Befestigungseinrichtung in einen Bereich der Gewebeprobe vorstehen, von dem die Abbildung angefertigt werden soll, um zu vermeiden, dass die Gewebeprobe auf der Aufnahme verdeckt ist.

Zweckmäßigerweise ist das Haltemittel derart vorgesehen, dass es bei Befestigung des Fixierungsmittels an der Grundplatte in einer Position zur Fixierung der Gewebeprobe von dem Steg schräg in Richtung zum Aufnahmeabschnitt hin, vorzugsweise in einem Winkel von 30° bis 70° zu einer durch den Aufnahmeabschnitt gebildeten Ebene, vorsteht.

In einer weiteren Ausführungsform der Erfindung ist das Fixierungsmittel zur gleichzeitigen Befestigung an der Grundplatte auf gegenüberliegenden Seiten des Aufnahmeabschnitts vorgesehen. Vorzugsweise ragt der Steg dann vollständig über den Aufnahmeabschnitt.

Zweckmäßigerweise ist das Fixierungsmittel derart geformt, dass es bei Anordnung der Vorrichtung derart, dass die Grundplatte, vorzugsweise senkrecht oder verkippt zur Vertikalen, auf einem Untergrund, z.B. einem Röntgentisch, steht, eine Stütze gegen Verkippen der Vorrichtung bildet. Vorteilhaft lässt sich die Vorrichtung in verschiedenen Positionen stabil aufstellen, damit sich Aufnahmen, insbesondere Röntgenaufnahmen, der Gewebeprobe in verschiedenen Richtungen anfertigen lassen. Insbesondere kann die Gewebeprobe sowohl in horizontaler als auch in vertikaler Lagerung radiologisch untersucht werden. Ein zusätzlicher Ständer zum Halten der Vorrichtung in der vertikalen Stellung ist nicht notwendig.

In einer weiteren Ausgestaltung der Erfindung ist das Fixierungsmittel derart geformt, dass es einen Ständer für die Vorrichtung bildet, mittels dessen sich die Vorrichtung ohne direkten Kontakt der Grundplatte zu dem Untergrund aufstellen lässt. Zweckmäßigerweise ist das Hilfsmittel derart geformt, dass es die Gewebeprobe allein, insbesondere ohne Unterstützung durch die Grundplatte, tragen kann. Dies erweist sich als besonders vorteilhaft, wenn sich die Vorrichtung derart auf dem Fixierungsmittel anordnen lässt, dass die Grundplatte ohne direkten Kontakt und parallel zu dem Untergrund angeordnet ist. Die Gewebeprobe wird dann durch ihre Gewichtskraft von der Grundplatte weggezogen, kann sich von dieser lösen und wird durch das Haltemittel gehalten. Die Fläche der Gewebeprobe, die der Grundplatte zugewandt ist, lässt sich dadurch unverfälscht und ohne Kontakt mit der Grundplatte auf der Aufnahme darstellen. Ferner ist es möglich, die Vorrichtung aus der letztgenannten Position in eine Stellung zu bringen, in der die Grundplatte vertikal auf dem Untergrund angeordnet ist. Da die Gewebeprobe bei entsprechendem Handling dann in der Position ohne Kontakt mit der Grundplatte verbleibt, lässt sich auch in dieser Stellung eine unverfälschte Aufnahme ohne Kontakt der Gewebeprobe mit der Grundplatte anfertigen.

In einer weiteren Ausführungsform der Erfindung ist die Grundplatte mit zumindest einem Standfuß versehen, der einen Anschlag zur Ausrichtung der Grundplatte an einem Rand eines Röntgentisches aufweist. Der Anschlag dient als Orientierung für die Anordnung der Grundplatte an einem vorderen Rand des Röntgentisches. Die Grundplatte ist bevorzugt an dem Rand anzuordnen, weil in dem Bereich, in dem sich die Grundplatte dann befindet, die Röntgenstrahlen vertikal verlaufen und sich verzerrungsfreie Röntgenaufnahmen erstellen lassen. Während es vorstellbar wäre, jeweils einen Anschlag für die Anordnung der Grundplatte in horizontaler Position oder in vertikaler Position auf dem Röntgentisch vorzusehen, ist der Standfuß vorzugsweise derart vorgesehen, dass der Anschlag sowohl für die horizontale als auch für die vertikale Anordnung gebildet ist.

Zweckmäßigerweise ist das Fixierungsmittel derart ausgebildet, dass es sich, vorzugsweise mittels der Befestigungseinrichtung, in einer Transportposition an der Grundplatte befestigen lässt, in der es flach auf der Grundplatte aufliegt. In der Transportposition sind der Steg und ggf. der Schenkel vorzugsweise parallel zu Rändern der Grundplatte angeordnet.
Die Form der Grundplatte und des Fixierungsmittels, insbesondere des Haltemittels, können derart aneinander angepasst sein, dass ein zum Angriff an der Gewebeprobe vorgesehenes Ende des Haltemittels in der Transportposition innerhalb der Gesamtheit aus Grundplatte und Fixierungsmittel angeordnet ist. Vorteilhaft bleiben die Enden der Haltemittel dadurch verborgen. Verletzungen an dem Ende des Haltemittels, die insbesondere auftreten können, wenn dieses spitz oder scharf ist, können vermieden werden. Ferner lässt sich vermeiden, dass eine für die Vorrichtung vorgesehene Verpackung durch das Ende beschädigt wird.

In einer weiteren Ausgestaltung der Erfindung sind zumindest zwei der Fixierungsmittel vorgesehen und die Fixierungsmittel lassen sich derart an der Grundplatte befestigen, dass sie jeweils von verschiedenen Seiten her an der Gewebeprobe angreifen können.

Zweckmäßigerweise ist der genannte Aufnahmeabschnitt durch ein offenes Gitter gebildet. Die offene Gitterstruktur erlaubt die Anfertigung einer direkten radiologischen Aufnahme (Röntgenbild) der Gewebeprobe und erlaubt darüber hinaus bei Lagerung in Formalin die Formalinfixierung der auf dem Aufnahmeabschnitt aufliegenden Unterseite (basale Fläche) der Gewebeprobe.
Vorteilhaft lassen sich darüber hinaus bei entsprechender Markierung von durch Hohlräume des Gitters gebildete Gitterzeilen und/oder-spalten Untersuchungsergebnisse der Gewebeprobe räumlich genau zuordnen. Das Gitter stellt dann in Verbindung mit der Markierung, die durch Buchstaben und/oder Zahlen gebildet sein kann, die vorzugsweise den jeweiligen Zeilen bzw. Spalten zugeordnet sind, eine Matrix zur Beschreibung der Lokalisation von Veränderungen innerhalb der Gewebeprobe (z.B. Mikrokalk, oder Tumor) dar.

In einer Ausführungsform der Erfindung ist der Aufnahmeabschnitt, insbesondere das Gitter, mit vorstehenden Pins zum Halten der Gewebeprobe versehen, um ein seitliches Abrutschen der Gewebeprobe zu verhindern. Die Pins sind zweckmäßigerweise an Stellen angeordnet, an denen sich Gitterstäbe des Gitters kreuzen.

Zweckmäßigerweise ist die Grundplatte, insbesondere auch die Gitterstäbe, und/ oder das Fixierungsmittel aus röntgenopakem Material gebildet.

In einer Ausgestaltung der Erfindung ist die Markierung durch Aussparungen in der Grundplatte und/oder in dem Fixierungsmittel gebildet. Die Aussparungen im Material der Grundplatte sind für Röntgenstrahlen im Vergleich zu dem röntgenopaken Material durchlässiger und dadurch auf dem erzeugten Röntgenbild erkennbar. Es versteht sich, dass die Markierung vorzugsweise in einer derartigen Größe vorgesehen wird, dass sie mit bloßem Auge und bei Abbildung auf dem Röntgenbild lesbar ist.

Vorzugsweise umfasst die Markierung zur Anzeige zumindest einer der topografischen Orientierungen, die die Gewebeprobe vor ihrer Entnahme im menschlichen oder tierischen Körper hatte, zumindest einen der Begriffe "medial", "lateral", "cranial", "caudal", "dorsal" oder "ventral" oder eine Abkürzung davon. Medial bedeutet zur Mitte des Körpers hin, lateral zur Seite des Körpers hin, cranial in Richtung zum Kopfes hin caudal nach unten hin, dorsal zum Rücken hin und ventral zur Vorderseite hin.

Nachfolgend ist die Erfindung anhand eines Ausführungsbeispiels unter Bezugnahme auf die beigefügten Figuren beschrieben. Es zeigen:
- Fig. 1 bis 6: eine erfindungsgemäße Vorrichtung in verschiedenen Positionen, und
- Fig. 7 bis 13: eine weitere erfindungsgemäße Vorrichtung in verschiedenen Positionen.

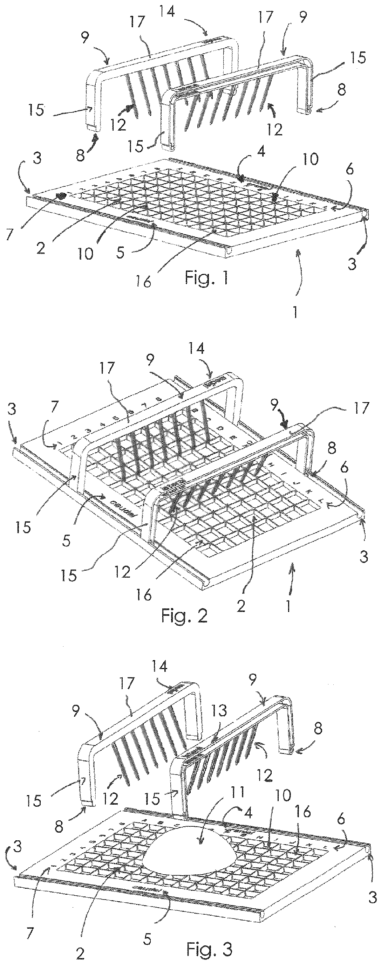

Eine erste erfindungsgemäße Vorrichtung für den Transport und/oder der Lagerung einer Gewebeprobe 11, die in den Fig. 1 bis 6 gezeigt ist, umfasst eine Grundplatte 1 und Fixierungsbrücken 9, mittels derer sich die Gewebeprobe 11 an der Grundplatte 1 festhalten lässt. Die Grundplatte 1 weist als Aufnahmeabschnitt für die Gewebeprobe 11 ein offenes Gitter 2 und an einander gegenüberliegenden seitlichen Rändern jeweils eine Befestigungsschiene 3 auf, die eine entlang des Randes gebildete Nut umfasst.

Durch Aussparungen 4,5 im Material, die mit bloßem Auge und auf einem die Vorrichtung abbildenden Röntgenbild erkennbar sind, sind neben den Befestigungsschienen 3 die Bezeichnungen "cranial" und "caudal" gebildet. Ebenfalls mit bloßem Auge und im Röntgenbild erkennbare weitere Aussparungen 6,7 sind in der Grundplatte 1 neben dem Gitter 2 jeweils eine Buchstabenreihe und eine Zahlenreihe ausgebildet, wobei die Zahlenreihe parallel zu den Befestigungsschienen 3 und die Buchstabenreihe senkrecht dazu angeordnet ist.

Das Gitter 2 der Grundplatte 1 weist an Überkreuzungen ihrer vertikalen und horizontalen Gitterstäbe in Fig. 2 gezeigte, nach oben vorstehende, feine Spitzen 10 auf, die ein seitliches Verrutschen des Gewebes 11 auf der Grundplatte 1 verhindern.

Jede der Fixierungsbrücken 9 umfasst einen Steg 17, an dem seitlich abgewinkelt Fixierungspins 12 befestigt sind, und an jedem Ende des Stegs 17 Schenkel 15, die mit ihren Enden 8 in die Befestigungsschienen 3 einsteckbar sind. Die Enden 8 der Schenkel 15 weisen eine gewölbte Form auf, sodass sie beim Einsetzen in die Nuten der Befestigungsschienen 3, die eine zu der Wölbung passende Form aufweisen, einrasten. Wie den Figuren zu entnehmen ist, können die Fixierungsbrücken 9 entlang der Ränder der Grundplatte 1, an denen die Befestigungsschienen 3 gebildet sind, in frei wählbaren Positionen an der Grundplatte befestigt werden, sodass die Positionen stufenlos ausgewählt werden können.

In den Stegen 17 der Fixierungsbrücken 9 sind auf der oberen Seite durch Aussparungen in Material - mit bloßen Augen und im Röntgenbild erkennbar - die Schriftzüge lateral 13 und medial 14 gebildet, um die topografische Orientierung der Gewebeprobe 11 anzuzeigen. Vorteilhaft lassen sich die Fixierungsbrücken 9 passend zur Anordnung der Gewebeprobe 11 auf der Grundplatte 1 anordnen, wozu die in den Fig. 1 bis 6 gezeigten Positionen getauscht werden können.

Wird das Gewebe 11 auf der Grundplatte 1 aufgelegt und mit Hilfe der Fixierungsbrücken 9 fixiert, kann es in einer vom Operateur vorgegebenen topographischen Orientierung transportiert und z.B. durch Anfertigung einer Röntgenaufnahme untersucht werden. Dazu wird die Gewebeprobe 11 hinsichtlich ihrer ursprünglichen topografischen Anordnung in dem menschlichen oder tierischen Körper, dem sie entnommen worden ist, auf der Grundplatte angeordnet und die Fixierungsbrücken 9 in den passenden Richtungen medial bzw. lateral derart auf der Grundplatte 1 befestigt, dass die Nadeln 12 in die Gewebeprobe 11 einstechen, die Schenkel 15 und die Stege 16 aber außerhalb eines Aufnahmebereichs für die Röntgenaufnahme angeordnet sind.

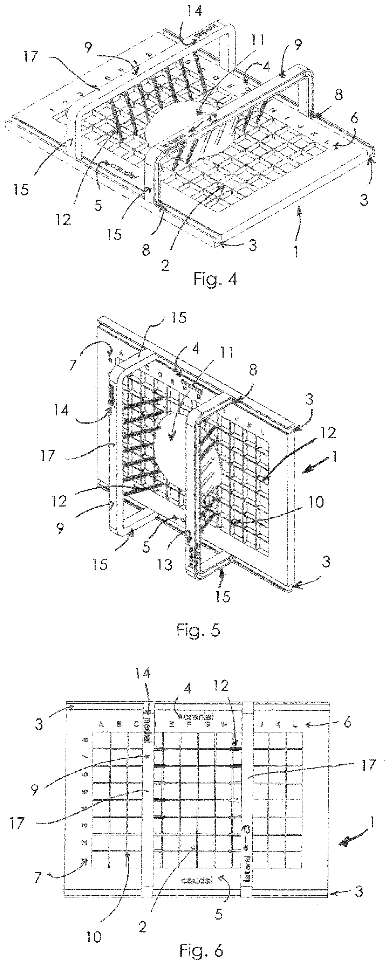

Wie insbesondere Fig. 5 zu entnehmen ist, kann die erfindungsgemäße Vorrichtung nicht nur derart auf einem Röntgentisch angeordnet werden, dass die Grundplatte 1 flach auf dem Röntgentisch aufliegt, sondern auch derart, dass die Grundplatte 1 senkrecht auf dem Röntgentisch steht. Dabei bilden die Schenkel 15 Stützen, die vermeiden, dass die Grundplatte 1 bei ihrer vertikalen Anordnung kippt. Durch die Möglichkeit zur Anordnung der Grundplatte 1 in horizontaler und vertikaler Position ist die Anfertigung von Aufnahmen in den Richtungen anterior/posterior (von vorne nach hinten) und cranial/caudal (von oben nach unten) möglich.

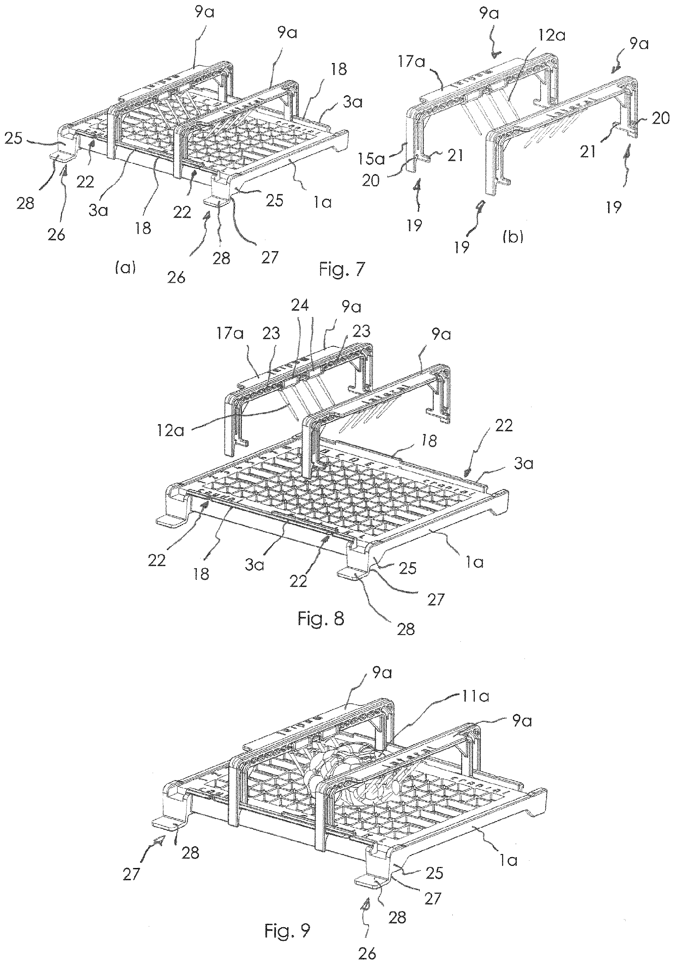

Es wird nun auf die Fig. 7 bis 13 Bezug genommen, in denen gleiche oder gleichwirkende Teile mit derselben Bezugszahl wie in den Fig. 1 bis 6 bezeichnet sind und der betreffenden Bezugszahl jeweils der Buchstabe a beigefügt ist.

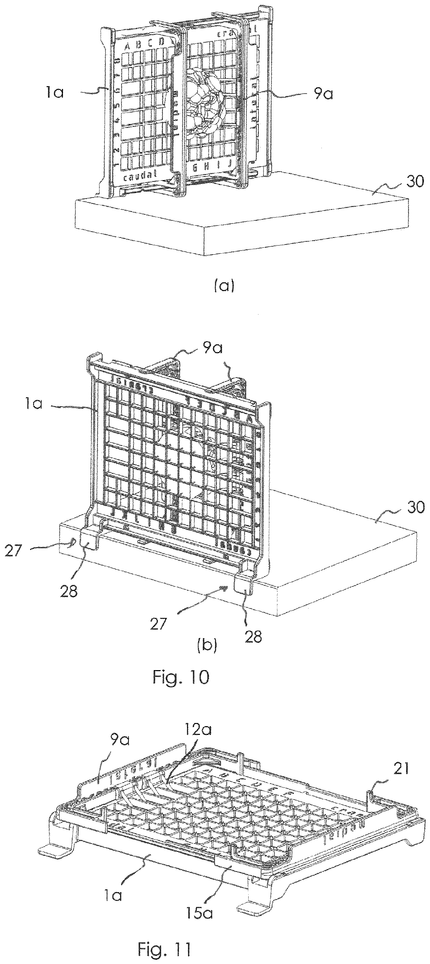

Eine weitere, in den Fig. 7 bis 13 gezeigte erfindungsgemäße Vorrichtung unterscheidet sich von denjenigen nach den Fig. 1 bis 6 insbesondere durch unterschiedliche Gestaltungen der Fixierungsbrücken 9a und Befestigungsschienen 3a. Wie insbesondere den Fig. 7a und 10a zu entnehmen ist, umfassen die Befestigungsschienen 3a je einen Steg 18, der von einem Rand einer Grundplatte 1a vorsteht. Zum Hintergreifen des Stegs 18 umfassen die Fixierungsbrücken 9a an Enden ihrer Schenkel 15a Rastelemente 19, die eine Rastnase 20 zum Hintergreifen des Stegs 18 sowie auf der gegenüberliegenden Seite des Stegs 18 angreifende Gegenhalter 21 aufweisen.
Wie Fig. 11 zu entnehmen ist, lassen sich die Fixierungsbrücken 9a auch in einer Transportposition an der Grundplatte 1a befestigen, indem die Stege 16a und die Schenkel 15a parallel zu den Rändern der Grundplatte 1 angeordnet werden. Damit sich die Fixierungsbrücken 9a auch in der Transportposition an der Grundplatte 1a befestigen lassen, sind in dem Steg 18 Ausnehmungen 22 gebildet, in die eine Rastnase 20 eingreifen kann.

Wie insbesondere Fig. 7b zu entnehmen ist, sind ferner in Stegen 17a der Fixierungsbrücken 9a Ausnehmungen 23 gebildet, in die sich Steckhalterungen 24 von Fixierungsnadeln 12a einstecken lassen, um die Position von Fixierungsnadeln 12a an der Fixierungsbrücke 9a an die jeweilige Form und Position der Gewebeprobe 11a anpassen zu können.

Ferner sind an Standfüßen 25, die an einem Rand der Grundplatte 1a vorgesehen sind, Anschläge 26 zur Anordnung der Grundplatte 1a an einem Röntgentisch 30 gebildet. Der Anschlag 26 umfasst einen ersten Anschlagsbereich 27, der für eine horizontale Anordnung der Grundplatte 1a auf dem Röntgentisch 30 vorgesehen ist sowie einen zweiten Anschlagsbereich 28 zur vertikalen Anordnung der Grundplatte 1 auf dem Röntgentisch 30 (vgl. Fig. 10).

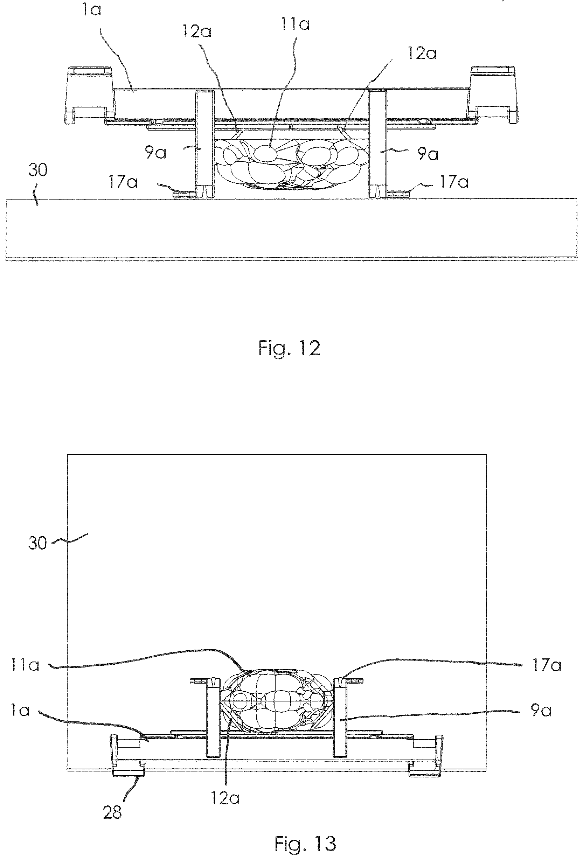

Wie Fig. 12 zu entnehmen ist, die die auf dem Röntgentisch 30 angeordnete Vorrichtung in einer Seitenansicht zeigt, kann die Vorrichtung auch auf den Stegen 17a der Fixierungsbrücken 9, d.h. umgekehrt zu der in Fig. 9 gezeigten Stellung, auf dem Röntgentisch 30 aufgestellt werden. Die Grundplatte 1a steht dann parallel und im Abstand zum Röntgentisch 30. Die Gewebeprobe 11a wird dann lediglich durch die Fixierungsnadeln 12a gehalten und liegt nicht mehr an der Grundplatte 1a an. Vorteilhaft lassen sich dann Aufnahmen des dem der Grundplatte 1a zugewandten Teils der Gewebeprobe 11a anfertigen, ohne dass dieser an der Grundplatte 1a liegt und dadurch verformt wird.

Die Gewebeprobe 11a kann, wie Fig. 13 in einer Draufsicht auf den Röntgentisch 30 zeigt, auch bei vertikaler Anordnung der Grundplatte 1a auf dem Röntgentisch 30 derart an der Vorrichtung befestigt werden, dass sie lediglich durch die Fixierungsnadeln 12a gehalten wird und nicht an der Grundplatte 1a anliegt.

## Patentansprüche

1. Vorrichtung zum Transport, zur Untersuchung und/oder zur Lagerung einer Gewebeprobe, die eine Grundplatte (1), welche einen zumindest teilweise röntgentransparenten Abschnitt (2) zur Aufnahme der Gewebeprobe umfasst und mit einer Markierung (4,5,6,7,14) zur Anzeige der topografischen Orientierung der Gewebeprobe versehen ist, und zumindest ein Mittel (9) zur Fixierung der Gewebeprobe auf dem Aufnahmeabschnitt (2) aufweist, das derart eingerichtet ist, dass es sich unter Fixierung der Gewebeprobe lösbar an einem Rand oder außerhalb des Aufnahmeabschnitts (2) an der Grundplatte (1) befestigen lässt, wobei die Vorrichtung vorgesehen ist derart, dass eine Position, in der sich das Fixierungsmittel (9) unter Fixierung der Gewebeprobe (11) an der Grundplatte (1) lösbar befestigen lässt, frei wählbar ist und das Fixierungsmittel (9) zu seiner Befestigung an der Grundplatte (1) mit einer Befestigungseinrichtung (8) versehen ist, mittels derer sich das Fixierungsmittel (9) lösbar in einer Halteposition zum Halten der Gewebeprobe stecken und/oder in der Halteposition einrasten lässt,
wobei das Fixierungsmittel (9) einen über den Aufnahmeabschnitt (2) ragenden Steg (17) und ein an dem Steg (17) angeordnetes, zum Aufnahmeabschnitt (2) hin vorstehendes und zum Angriff an der Gewebeprobe vorgesehenes Haltemittel (12) umfasst.

2. Vorrichtung nach Anspruch 1,
wobei die Grundplatte (1) eine Befestigungsschiene (3), an der sich das Fixierungsmittel (9) anordnen lässt, aufweist.

3. Vorrichtung nach Anspruch 1 oder 2,
wobei die Befestigungseinrichtung (8) zu Ihrer Befestigung an der Befestigungsschiene (3) vorgesehen ist.

4. Vorrichtung nach einem der Ansprüche 1 bis 3,
wobei der Steg (17) mit zumindest einem mit der Befestigungseinrichtung versehenen Schenkel (18) verbunden ist.

5. Vorrichtung nach einem der Ansprüche 1 bis 4,
wobei das Haltemittel (12) einen Stift oder/und eine Nadel aufweist.

6. Vorrichtung nach einem der Ansprüche 1 bis 5,
wobei das Haltemittel (12) von dem Steg (17) schräg in Richtung zum Aufnahmeabschnitt (2) hin, vorzugsweise in einem Winkel von 30° bis 70° zu einer durch den Aufnahmeabschnitt (2) gebildeten Ebene, angeordnet ist.

7. Vorrichtung nach Anspruch 6,
wobei das Fixierungsmittel (9) zur gleichzeitigen Befestigung an der Grundplatte (1) auf gegenüberliegenden Seiten des Aufnahmeabschnitts (2) vorgesehen ist.

8. Vorrichtung nach einem der Ansprüche 1 bis 7,
wobei das Fixierungsmittel (9) derart geformt ist, dass es bei vertikaler Anordnung der Grundplatte (1) auf einem Untergrund, z.B. einem Röntgentisch, eine Stütze gegen Verkippen der Vorrichtung bildet.

9. Vorrichtung nach einem der Ansprüche 1 bis 8,
wobei zumindest zwei der Fixierungsmittel (9) vorgesehen sind und sich die Fixierungsmittel (9) derart an der Grundplatte (1) befestigen lassen, dass sie jeweils von verschiedenen Seiten her an der Gewebeprobe angreifen können.

10. Vorrichtung nach einem der Ansprüche 1 bis 9,
wobei der Aufnahmeabschnitt (2) durch ein, vorzugsweise offenes, Gitter gebildet ist.

11. Vorrichtung nach einem der Ansprüche 1 bis 10,
wobei der Aufnahmeabschnitt (2), vorzugsweise das Gitter, mit vorstehenden Pins (10) zum Halten der Gewebeprobe versehen ist, wobei die Pins (10) vorzugsweise an Stellen, an denen sich Gitterstäbe (16) des Gitters kreuzen, angeordnet sind.

12. Vorrichtung nach Anspruch 11,
wobei die Markierung (4,5,6,7,14) durch eine Aussparung in der Grundplatte (1) und/oder in dem Fixierungsmittel (9) gebildet ist.

13. Vorrichtung nach einem der Ansprüche 1 bis 12,
wobei die Grundplatte (1), insbesondere das Gitter, und/oder das Fixierungsmittel (9) aus röntgenopakem Material gebildet ist.

14. Vorrichtung nach einem Ansprüche 1 bis 13,
wobei sich das Fixierungsmittel (9) in einer Transportposition an der Grundplatte (1) befestigen lässt, in der es flach auf der Grundplatte (1) angeordnet ist.

## Claims

1. Device for transporting, examining and/or storing a tissue sample, having a base plate (1) which comprises an at least partially radioparent portion (2) for receiving the tissue sample and which is provided with a marking (4, 5, 6, 7, 14) for indicating the topographic orientation of the tissue sample, and at least one means (9) for fixing the tissue sample on the receiving portion (2), which means (9) is set up in such a way that, on fixing the tissue sample, it can be fastened releasably to the base plate (1) at an edge of or outside of the receiving portion (2), wherein the device is provided in such a way that a position in which the fixing means (9) can be releasably fastened to the base plate (1) when fixing the tissue sample (11) is freely selectable, and, for fastening the fixing means (9) to the base plate (1), said fixing means (9) is provided with a fastening mechanism (8) with which the fixing means (9) can be plugged releasably into a holding position for holding the tissue sample and/or can be locked in the holding position, wherein the fixing means (9) comprises a web (17), protruding over the receiving portion (2), and a holding means (12) arranged on the web (17), protruding towards the receiving portion (2) and provided for engaging on the tissue sample.

2. Device according to Claim 1, wherein the base plate (1) has a fastening rail (3) on which the fixing means (9) can be arranged.

3. Device according to Claim 1 or 2, wherein the fastening mechanism (8) is provided for fastening on the fastening rail (3).

4. Device according to one of Claims 1 to 3, wherein the web (17) is connected to at least one leg (18) provided with the fastening mechanism.

5. Device according to one of Claims 1 to 4, wherein the holding means (12) has a pin and/or a needle.

6. Device according to one of Claims 1 to 5, wherein the holding means (12) is arranged extending obliquely away from the web (17) in the direction of the receiving portion (2), preferably at an angle of 30° to 70° with respect to a plane formed by the receiving portion (2).

7. Device according to Claim 6, wherein the fixing means (9) is provided for fastening to the base plate (1) simultaneously on opposite sides of the receiving portion (2).

8. Device according to one of Claims 1 to 7, wherein the fixing means (9) is shaped in such a way that it forms a prop to prevent tipping of the device when the base plate (1) is arranged vertically on a support, e.g. an X-ray table.

9. Device according to one of Claims 1 to 8, wherein at least two of the fixing means (9) are provided, and the fixing means (9) can be fastened to the base plate (1) in such a way that they can each engage the tissue sample from different sides.

10. Device according to one of Claims 1 to 9, wherein the receiving portion (2) is formed by a preferably open grid.

11. Device according to one of Claims 1 to 10, wherein the receiving portion (2), preferably the grid, is provided with protruding pins (10) for holding the tissue sample, wherein the pins (10) are preferably arranged at locations at which grid bars (16) of the grid intersect.

12. Device according to Claim 11, wherein the marking (4, 5, 6, 7, 14) is formed by a recess in the base plate (1) and/or in the fixing means (9).

13. Device according to one of Claims 1 to 12, wherein the base plate (1), in particular the grid, and/or the fixing means (9) is formed from radiopaque material.

14. Device according to one of Claims 1 to 13, wherein the fixing means (9) can be fastened to the base plate (1) in a transport position in which it is arranged flat on the base plate (1).

## Revendications

1. Dispositif pour le transport, pour l'analyse et/ou pour le stockage d'un échantillon tissulaire, qui comprend une plaque de base (1), qui comprend une section (2) au moins partiellement transparente aux rayons X, destinée à recevoir l'échantillon tissulaire et qui est pourvue d'un marquage (4,5,6,7,14) pour indiquer l'orientation topographique de l'échantillon tissulaire, et au moins un moyen (9) pour fixer l'échantillon tissulaire sur la section de réception (2), qui est conçu de manière telle qu'il peut être fixé de manière amovible, tout en fixant l'échantillon tissulaire, à un bord ou à l'extérieur de la section de réception (2) à la plaque de base (1), le dispositif étant conçu de manière telle qu'une position dans laquelle le moyen de fixation (9) peut être fixé de manière amovible, tout en fixant l'échantillon tissulaire (11) à la plaque de base (1), peut être choisie librement et le moyen de fixation (9) étant pourvu, pour sa fixation à la plaque de base (1), d'un dispositif de fixation (8) au moyen duquel le moyen de fixation (9) peut être introduit de manière amovible dans une position de retenue pour retenir l'échantillon tissulaire et/ou être encliqueté de manière amovible dans la position de retenue, le moyen de fixation (9) comprenant une barre (17) surplombant la section de réception (2) et un moyen de retenue (12) disposé sur la barre (17), dépassant vers la section de réception (2) et destiné à venir en prise avec l'échantillon tissulaire.

2. Dispositif selon la revendication 1, la plaque de base (1) présentant un rail de fixation (3) sur lequel le moyen de fixation (9) peut être agencé.

3. Dispositif selon la revendication 1 ou 2, le dispositif de fixation (8) étant prévu pour sa fixation au rail de fixation (3).

4. Dispositif selon l'une quelconque des revendications 1 à 3, la barre (17) étant reliée à au moins une branche (18) pourvue du dispositif de fixation.

5. Dispositif selon l'une quelconque des revendications 1 à 4, le moyen de retenue (12) présentant une tige et/ou une aiguille.

6. Dispositif selon l'une quelconque des revendications 1 à 5, le moyen de retenue (12) étant agencé à partir de la tige (17) en biais dans la direction vers la section de réception (2), de préférence sous un angle de 30° à 70° par rapport à un plan formé par la section de réception (2).

7. Dispositif selon la revendication 6, le moyen de fixation (9) étant conçu pour la fixation à la plaque de base (1) simultanément aux côtés opposés de la section de réception (2).

8. Dispositif selon l'une quelconque des revendications 1 à 7, le moyen de fixation (9) étant formé de manière telle que lors de l'agencement vertical de la plaque de base (1) sur un support, par exemple une table à rayons X, il forme un appui contre le renversement du dispositif.

9. Dispositif selon l'une quelconque des revendications 1 à 8, au moins deux des moyens de fixation (9) étant prévus et les moyens de fixation (9) pouvant être fixés à la plaque de base (1) de manière telle qu'ils peuvent venir en prise avec l'échantillon tissulaire à chaque fois depuis différents côtés.

10. Dispositif selon l'une quelconque des revendications 1 à 9, la section de réception (2) étant formée par une grille, de préférence ouverte.

11. Dispositif selon l'une quelconque des revendications 1 à 10, la section de réception (2), de préférence la grille, étant pourvue de broches (10) qui dépassent, destinées à retenir l'échantillon tissulaire, les broches (10) étant de préférence agencées en des endroits dans lesquels des barreaux (16) de la grille se croisent.

12. Dispositif selon la revendication 11, le marquage (4,5,6,7,14) étant formé par un évidement dans la plaque de base (1) et/ou dans le moyen de fixation (9).

13. Dispositif selon l'une quelconque des revendications 1 à 12, la plaque de base (1), en particulier la grille, et/ou le moyen de fixation (9) étant formés d'un matériau opaque aux rayons X.

14. Dispositif selon l'une quelconque des revendications 1 à 13, le moyen de fixation (9) pouvant être fixé dans une position de transport, dans laquelle il est agencé à plat sur la plaque de base (1), à la plaque de base (1).
